# EUROPÄISCHE PATENTSCHRIFT

(11) **EP 4 380 499 B1**
(45) Veröffentlichungstag und Bekanntmachung des Hinweises auf die Patenterteilung: **26.03.2025**
(21) Anmeldenummer: 22754012.7
(22) Anmeldetag: 15.07.2022
(51) Int. Cl.: A61F 2/16

(54) **BEHÄLTERVORRICHTUNG MIT EINEM EINE INTRAOKULARLINSE AUFWEISENDEN OPHTHALMOLOGISCHEN INJEKTOR**
CONTAINER DEVICE COMPRISING AN OPHTHALMOLOGICAL INJECTOR THAT CONTAINS AN INTRAOCULAR LENS
DISPOSITIF DE RÉCIPIENTS COMPRENANT UN INJECTEUR OPHTALMOLOGIQUE PRÉSENTANT UNE LENTILLE INTRAOCULAIRE

(30) Priorität: 02.08.2021 DE 102021208333
(43) Veröffentlichungstag der Anmeldung: 12.06.2024
(73) Patentinhaber: Carl Zeiss Meditec AG, 07745 Jena (DE)
(72) Erfinder: MÜLLER, Marco, 10437 Berlin (DE); WOLFSTEIN, Andre, 13507 Berlin (DE)
(74) Vertreter: Carl Zeiss AG - Patentabteilung
(86) Internationale Anmeldenummer: PCT/EP2022/069830
(87) Internationale Veröffentlichungsnummer: WO 2023/011886

(56) Entgegenhaltungen:
- WO-A1-2018/015784
- WO-A1-2021/062568
- AT-U1- 12 360
- US-A1- 2007 250 068
- US-A1- 2015 114 855

## Beschreibung

Die Erfindung betrifft eine Behältervorrichtung, welche einen eine Intraokularlinse aufweisenden ophthalmologischen Injektor aufweist.

Intraokularlinsen (IOLs) werden typischerweise in zwei Gruppen aufgeteilt: hydrophobe Intraokularlinsen, welche weniger als 1 Prozent Wasser absorbieren und hydrophile Intraokularlinsen, welche mehr als 1 Prozent Wasser absorbieren. Hydrophobe Intraokularlinsen können bei Raumtemperatur gefaltet werden, wobei ihre Faltbarkeit durch ihre chemische Zusammensetzung und nicht aufgrund des Wassers, welches als Weichmacher fungiert, sichergestellt ist. Im Gegensatz hierzu können hydrophile Linsen, welche typischerweise einen Anteil von etwa 25 Gewichtsprozent Wasser enthalten, aufgrund des als Weichmacher dienenden absorbierten Wassers bei Raumtemperatur gefaltet werden.

In der Fachwelt geht man davon aus, dass hydrophile Intraokularlinsen während der Lagerung in Wasser oder physiologischer Kochsalzlösung getränkt werden müssen, um eine ausreichende Hydration, welche zum Falten bei Raumtemperatur benötigt wird, sicherzustellen. Aufgrund dessen werden solche Intraokularlinsen so verpackt, dass sie sich in einem derartigen Fluid befinden, in welchem sie bis zu fünf Jahre vor ihrer Implantation verweilen können, ohne in dieser Zeit ihre optischen, mechanischen und chemischen Eigenschaften zu verändern.

Intraokularlinsen werden typischerweise durch eine Implantationsvorrichtung, welche vom Fachmann gewöhnlich als ophthalmologischer Injektor bezeichnet wird, während einer Kataraktoperation implantiert. Vorzugsweise wird die Intraokularlinse in einem vorgespannten Zustand im Injektor gelagert, um das Risiko von Fehloperationen aufgrund von Bedienfehlern zu minimieren. Die Lagerung der Intraokularlinse erfolgt in einer feuchten Umgebung, um eine ausreichende Hydration der Intraokularlinse sicherzustellen.

Dazu wird mindestens ein Teil des ophthalmologischen Injektors, in welchem sich die bevorzugt vorgespannte Intraokularlinse befindet, in einem mit Fluid gefüllten Behälter gelagert. Als Fluid kann hier Wasser oder Balanced Salt Solution (BSS) verwendet werden.

Solche Systeme sind zum Beispiel in US 2007 / 0 250 068 A1, WO 2010 / 105 678 A1, WO 2013 / 159 045 A1, US 2011 / 0 245 840 A1 und WO 2021 / 062 568 A1 beschrieben.

Allerdings weisen solcherlei Systeme einige Nachteile auf. Zum einen können die sterilen Handschuhe eines Benutzers beim Herausnehmen des Injektors aus dem Wasserbad feucht werden, wodurch sich ein Kontaminationsrisiko erhöhen kann, da Bakterien von feuchten Oberflächen leichter übertragen werden können. Zum anderen wird die Handhabung eines Injektors durch feuchte Hände erschwert, was zu Problemen während der Operation führen kann. Weiter kann das Fluid aus dem System herausschwappen, was zur Folge hätte, dass die Operationsumgebung feucht wird.

In diesem Zusammenhang wird auf US 5 616 184 A, US 2009 / 0 057 167 A1, US 2002 / 0 029 981 A1 und DE 10 2014 005 719 A1 verwiesen.

Eine Behältervorrichtung gemäß der Preambel von Anspruch 1 ist aus dem Dokument US 2015/114855 bekannt.

Es ist eine Aufgabe der Erfindung, eine Behältervorrichtung zu schaffen, welche es ermöglicht, den die Intraokularlinse enthaltenden ophthalmologischen Injektor in geeigneter Weise aus dem mit Fluid gefüllten Behälter zu entnehmen, ohne dass das Fluid aus der Behältervorrichtung entweicht und somit die Umgebung der Behältervorrichtung trocken bleibt. Zusätzlich soll erreicht werden, dass ein Benutzer ein möglichst geringes Kontaminationsrisiko darstellt, nachdem er den ophthalmologischen Injektor aus der Behältervorrichtung entnommen hat. Ebenfalls ist es eine Aufgabe der Erfindung, eine gattungsgemäße Behältervorrichtung zu schaffen, welche eine höhere Sicherheit aufweist, falls die Behältervorrichtung versehentlich umgestoßen wird, wobei erreicht werden soll, dass das Fluid in der Behältervorrichtung zum größten Teil verbleibt und somit die Operationsumgebung außerhalb der Behältervorrichtung im Wesentlichen trocken bleibt. Ferner soll erreicht werden, dass die gattungsgemäße Behältervorrichtung in einfacher und kostengünstiger Weise zu fertigen ist und aus möglichst wenigen Einzelteilen besteht.

Die Aufgabe wird durch den Gegenstand des Anspruchs 1 gelöst. Vorteilhafte Weiterbildungen der Erfindung sind Gegenstand der Unteransprüche.

Die erfindungsgemäße Behältervorrichtung weist einen ophthalmologischen Injektor, welcher eine Intraokularlinse aufweist, sowie einen ersten Behälter auf, der zumindest teilweise mit einem Fluid gefüllt ist, wobei die Intraokularlinse von dem Fluid umgeben ist, und der erste Behälter mit einem Deckel versehen ist, welcher den ersten Behälter fluiddicht verschließt und lösbar mit dem ersten Behälter verbunden ist. Die Behältervorrichtung weist zusätzlich einen zweiten Behälter auf, der mit dem ersten Behälter gekoppelt ist. Zwischen dem ersten Behälter und dem zweiten Behälter ist ein fluiddichter Verschluss vorgesehen, wobei der Deckel mittels einer Kopplungsvorrichtung mit dem Verschluss gekoppelt ist, und bei einem Lösen des Deckels vom ersten Behälter der Verschluss mittels der Kopplungsvorrichtung eingerichtet ist, von einer Geschlossen-Position in eine Offen-Position zu gelangen, sodass das Fluid von dem ersten Behälter in den zweiten Behälter strömen kann.

In der Behältervorrichtung befindet sich somit ein Injektor, welcher eine Intraokularlinse beinhaltet. Zusätzlich weist die Behältervorrichtung einen ersten Behälter auf, welcher zumindest teilweise mit einem Fluid befüllt ist. Der erste Behälter ist mit einem Deckel ausgestattet, welcher den ersten Behälter fluiddicht verschließt und lösbar mit dem ersten Behälter verbunden ist. Der Deckel bewirkt also, dass das Fluid bei verschlossenem Deckel nicht aus dem ersten Behälter entweichen kann. Allerdings kann der Deckel von dem ersten Behälter gelöst werden, wodurch der erste Behälter geöffnet wird.

Die Intraokularlinse ist von dem im ersten Behälter befindlichen Fluid umgeben. Dies ist unerlässlich, da hydrophile Intraokularlinsen während der Lagerung in einem hydratisierten Zustand gehalten werden müssen, wodurch sichergestellt ist, dass die Intraokularlinse faltbar bleibt. Der Injektor kann also somit teilweise, aber auch in seiner gesamten Ausgestaltung vom Fluid umgeben sein. Weiter kann sich der Injektor teilweise, aber auch in seiner gesamten Ausgestaltung in dem ersten Behälter befinden.

Darüber hinaus weist die Behältervorrichtung einen zweiten Behälter auf, welcher mit dem ersten Behälter gekoppelt ist. Der erste Behälter und der zweite Behälter sind somit miteinander verbunden.

Zwischen dem ersten Behälter und dem zweiten Behälter befindet sich gemäß der Erfindung ein fluiddichter Verschluss, wobei der Deckel mittels einer Kopplungsvorrichtung mit dem Verschluss verbunden ist. Der Verschluss ist eingerichtet, bei einem Lösen des Deckels von dem ersten Behälter, mittels der Kopplungsvorrichtung von einer Geschlossen-Position in eine Offen-Position zu gelangen, sodass das Fluid von dem ersten Behälter in den zweiten Behälter strömen kann. Bei verschlossenem Deckel befindet sich das Fluid also in dem ersten Behälter. Der Verschluss zwischen dem ersten Behälter und dem zweiten Behälter ist geschlossen. Sobald der Deckel gelöst wird, öffnet sich mittels der Kopplungsvorrichtung der Verschluss, sodass das Fluid in den zweiten Behälter strömen kann. Anschließend befindet sich kein Fluid mehr in dem ersten Behälter.

Damit wird erreicht, dass das Fluid von dem ersten Behälter in den zweiten Behälter verlagert wird. Der ophthalmologische Injektor, welcher die Intraokularlinse enthält, kann aus dem ersten Behälter entnommen werden, ohne dass der Benutzer in das Fluid greifen muss. Ferner weist ein Benutzer, nachdem er den ophthalmologischen Injektor aus der erfindungsgemäßen Behältervorrichtung entnommen hat, ein geringes Kontaminationsrisiko auf. Selbst wenn die Behältervorrichtung versehentlich umgestoßen wird, wird hierdurch sichergestellt, dass das Fluid in der Behältervorrichtung zum größten Teil verbleibt und die Umgebung im Wesentlichen trocken bleibt. Durch die wenigen Bauteile kann die erfindungsgemäße Behältervorrichtung einfach und kostengünstig hergestellt werden.

Das Fluid kann unter anderem Wasser oder eine Balanced Salt Solution (BSS) sein.

Die Kopplungsvorrichtung kann unter anderem eine Schnur, ein Band, ein Bindfaden, ein Seil, oder eine zugfeste Verbindung in Form eines Drahtes sein. Die Kopplungsvorrichtung kann auch ein Teil des ersten Behälters sein, welcher mit dem Deckel verbunden ist. Auch der fluiddichte Verschluss kann ein Teil des ersten Behälters sein. Eine Wand des ersten Behälters, welche eine Seitenwand oder eine Bodenwand des ersten Behälters ist, kann einen ersten Wandbereich mit einer ersten Dicke und einen zweiten Wandbereich mit einer zweiten Dicke aufweisen. Der fluiddichte Verschluss kann zumindest teilweise durch einen Teil der Wand des ersten Behälters ausgebildet sein, wobei der Verschluss durch den zweiten Wandbereich zumindest teilweise begrenzt ist. Die zweite Dicke ist kleiner als die erste Dicke, sodass um den Verschluss eine Sollbruchstelle vorgesehen ist. Wenn der Deckel geöffnet wird, überträgt sich die Zugkraft mittels der Kopplungsvorrichtung auf den Verschluss, welcher an dem Teil der Wand des ersten Behälters aufbricht, welcher die zweite Dicke aufweist, sodass der Verschluss von einer Geschlossen-Position in eine Offen-Position gelangt. Dadurch ist es möglich, dass das Fluid in den zweiten Behälter abfließen kann.

Wenn der fluiddichte Verschluss an einer Seitenwand angebracht ist, kann die Behältervorrichtung sehr kompakt gebaut werden. Die Bodenwand kann dann so klein ausgeführt sein, dass darauf nur der Injektor Platz findet und keine zusätzliche Fläche für den Verschluss an der Bodenwand vorgesehen sein muss.

Gemäß einer Weiterbildung der Erfindung weist der fluiddichte Verschluss eine Fläche auf, wobei die Fläche des Verschlusses eine Größe in einem Bereich von 50 % bis 20 % der Fläche einer Wand der Behältervorrichtung besitzt, wobei die Wand eine Seitenwand oder eine Bodenwand ist. Die Fläche des Verschlusses kann auch eine Größe in einem Bereich von 20 % bis 10 % der Fläche einer Wand der Behältervorrichtung besitzen oder eine Größe in einem Bereich von 10 % bis 1 % der Fläche einer Wand der Behältervorrichtung oder eine Größe in einem Bereich von 1 % bis 0,2 % der Fläche einer Wand der Behältervorrichtung, besitzen. Hierdurch wird sichergestellt, dass das Fluid bei versehentlichem Umstoßen der Behältervorrichtung, wenn sich der Verschluss in der Offen-Position befindet, zum Teil in dem zweiten Behälter verbleibt oder nur verzögert auslaufen kann. Dadurch wird die Sicherheit erhöht, dass bei einem solchen versehentlichen Umstoßen der Behältervorrichtung die Umgebung der Behältervorrichtung im Wesentlichen trocken bleibt. Darüber hinaus wird sichergestellt, dass das Fluid ausreichend schnell von dem ersten Behälter in den zweiten Behälter fließen kann.

Bevorzugt ist der fluiddichte Verschluss als ein Einwegeventil ausgebildet. Hierfür wird in vorteilhafter Weise erreicht, dass das Fluid nicht mehr von dem zweiten Behälter in den ersten Behälter zurückfließen kann. Das Fluid verbleibt also aufgrund des Einwegeventils vollständig in dem zweiten Behälter. Der Vorteil hierfür liegt weiter darin, dass dadurch keine aktive Tätigkeit durch den Benutzer der erfindungsgemäßen Behältervorrichtung erforderlich ist, um das Fluid separat von dem Injektor einzuschließen. Dies erhöht nochmals die Sicherheit im Umgang mit der Behältervorrichtung, da eine hohe Sicherheit besteht, dass kein Fluid vom zweiten Behälter in eine Operationsumgebung entweichen kann.

Gemäß einer Weiterbildung der Erfindung liegt in dem ersten Behälter ein erster Druck und in dem zweiten Behälter ein zweiter Druck vor. In einer bevorzugten Ausführungsform ist der zweite Druck niedriger als der erste Druck, wodurch erreicht wird, dass beim Öffnen des Deckels das Fluid von dem ersten Behälter schneller in den zweiten Behälter strömen kann, als wenn in beiden Behältern der gleiche Druck herrschen würde. Hierdurch ist es möglich, dass der Benutzer der Behältervorrichtung relativ schnell den Injektor aus dem ersten Behälter entnehmen kann, ohne in das Fluid greifen zu müssen.

In einer weiteren Ausführungsform weist mindestens eine Wand des zweiten Behälters, welche eine Seitenwand oder eine Bodenwand des zweiten Behälters ist, mindestens eine Zone auf, welche eine niedrigere Federkonstante als ein diese Zone umgebender Bereich der Wand aufweist. Hierdurch wird in vorteilhafter Weise erreicht, dass sich bei Erhöhung des Druckes in dem zweiten Behälter das Volumen des zweiten Behälters relativ zu dem Volumen des ersten Behälters vergrößern kann. Der dadurch resultierende positive Effekt ist, dass ein größerer Anteil des Fluids in den zweiten Behälter gelangen kann, auch wenn das Volumen des zweiten Behälters aufgrund von einzusparendem Bauraum zu Transportzwecken bei geschlossenem Deckel zunächst möglichst klein gehalten wird.

Bevorzugt weist der zweite Behälter eine fluidabsorbierende Vorrichtung auf. Die fluidabsorbierende Vorrichtung kann unter anderem ein Schwamm, Silikagel, ein Trocknungsmittel oder ein anderes wasseraufnehmendes Mittel sein. Hierdurch wird in vorteilhafter Weise erreicht, dass das Fluid in dem zweiten Behälter so aufgenommen werden kann, dass es nicht mehr frei beweglich ist. Es wird zum Beispiel verhindert, dass das Fluid in dem zweiten Behälter bei einer räumlichen Verlagerung einen schwankenden Fluidpegel aufweist. Dies verringert auch die Gefahr, dass das Fluid bei einer stärkeren Bewegung im Raum aus dem zweiten Behälter in dessen Umgebung ausströmen kann.

Ferner ist es möglich, dass der Deckel den zweiten Behälter fluiddicht verschließt und lösbar mit dem zweiten Behälter verbunden ist. Der erste Behälter befindet sich somit in dem zweiten Behälter, wodurch in vorteilhafter Weise die Behältervorrichtung relativ kompakt ist.

Bevorzugt ist die Kopplungsvorrichtung ein Teil des ersten Behälters, sodass eine einstückige Konstruktion der Kopplungsvorrichtung mit dem ersten Behälter vorliegt. Ein Ende dieser Kopplungsvorrichtung ist mit dem Verschluss verbunden, sodass durch Aufbringen einer Zugkraft auf die Kopplungsvorrichtung der Verschluss, welcher ebenfalls einen Teil des ersten Behälters bildet, aufbrechen kann.

Weitere Vorteile und Merkmale werden mit Bezug auf die nachfolgenden Figuren erklärt, in welchen zeigen:
- Fig. 1: eine schematische Darstellung einer Querschnittsansicht von einer Behältervorrichtung gemäß einer ersten Ausführungsform der Erfindung in einer Geschlossen-Position;
- Fig. 2: eine schematische Darstellung der Querschnittsansicht von der Behältervorrichtung gemäß der ersten Ausführungsform in einer Offen-Position;
- Fig. 3: eine schematische Darstellung einer Draufsicht von der Behältervorrichtung gemäß der ersten Ausführungsform;
- Fig. 4: eine schematische Darstellung einer Querschnittsansicht von der Behältervorrichtung gemäß einer zweiten Ausführungsform der Erfindung in einer Geschlossen-Position;
- Fig. 5: eine schematische Darstellung der Querschnittsansicht von der Behältervorrichtung gemäß der zweiten Ausführungsform in einer Offen-Position;
- Fig. 6: eine schematische Darstellung einer Querschnittsansicht von der Behältervorrichtung gemäß einer dritten Ausführungsform in einer Geschlossen-Position;
- Fig. 7: eine schematische Darstellung einer Querschnittsansicht von der Behältervorrichtung gemäß der dritten Ausführungsform in einer Offen-Position; und
- Fig. 8: eine schematische Darstellung einer Draufsicht auf den ersten Behälter mit einem ersten Wandbereich und einem zweiten Wandbereich.

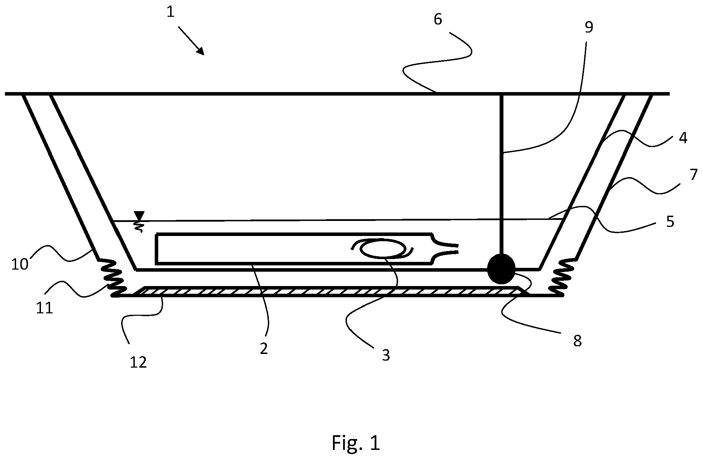

In Fig. 1 ist eine schematische Darstellung einer Querschnittsansicht von einer Behältervorrichtung 1 gemäß einer ersten Ausführungsform in einer Geschlossen-Position dargestellt. Die Behältervorrichtung 1 weist einen ophthalmologischen Injektor 2 auf, in welchem sich eine Intraokularlinse 3 befindet. Die Intraokularlinse 3 befindet sich bevorzugt in einem vorgespannten Zustand.

Die Behältervorrichtung 1 weist weiter einen ersten Behälter 4 auf, in welchem sich der ophthalmologische Injektor 2 befindet. Der erste Behälter 4 ist zumindest teilweise mit einem Fluid 5 gefüllt, wobei die Intraokularlinse 3 von dem Fluid 5 vollständig umgeben ist. Hierdurch ist sichergestellt, dass sich die Intraokularlinse 3 während der Lagerung in einem hydratisierten Zustand befindet. Eine hydrophile Intraokularlinse 3 bleibt somit gut faltbar. Es ist ersichtlich, dass der Injektor 2 teilweise, aber auch in seiner gesamten Ausgestaltung von dem Fluid 5 umgeben sein kann.

Der erste Behälter 4 weist einen Deckel 6 auf, welcher den ersten Behälter 4 fluiddicht verschließt und lösbar mit dem ersten Behälter 4 verbunden ist. Durch den Deckel 6 ist also sichergestellt, dass das Fluid 5 aus dem ersten Behälter 4 bei verschlossenem Deckel 6 nicht entweichen kann. Der Deckel 6 kann von einem Benutzer von dem ersten Behälter 4 mindestens teilweise so gelöst werden, dass der erste Behälter 4 mindestens in einem Teilbereich geöffnet wird. Bei einem derart geöffnetem Deckel 6 kann der ophthalmologische Injektor 2, in welchem sich die Intraokularlinse 3 befindet, aus dem ersten Behälter 4 entnommen werden.

Die Behältervorrichtung 1 weist weiter einen zweiten Behälter 7 auf, der mit dem ersten Behälter 4 gekoppelt ist. Der erste Behälter 4 ist also mit dem zweiten Behälter 7 verbunden. In der in Fig. 1 gezeigten ersten Ausführungsform befindet sich der erste Behälter 4 in dem zweiten Behälter 7, wodurch die Behältervorrichtung 1 relativ kompakt ausgebildet ist. In dieser Ausführungsform verschließt der Deckel 6 zusätzlich zu dem ersten Behälter 4 auch den zweiten Behälter 7 fluiddicht. Außerdem ist der Deckel 6 ebenfalls mit dem zweiten Behälter 7 lösbar verbunden.

Zwischen dem ersten Behälter 4 und dem zweiten Behälter 7 befindet sich ein fluiddichter Verschluss 8. Durch den fluiddichten Verschluss 8 wird sichergestellt, dass das Fluid 5 nicht von dem ersten Behälter 4 in den zweiten Behälter 7 strömen kann, falls sich der Verschluss 8 in einer Geschlossen-Position befindet.

Der zweite Behälter 7 weist mindestens eine Wand 10, insbesondere eine Seitenwand, auf, in welcher jeweils mindestens eine Zone 11 existiert, welche eine niedrigere Federkonstante aufweist als ein diese Zone 11 umgebender Bereich der Wand 10. Die Zone 11 ist somit elastischer als der Bereich, welcher diese Zone 11 umgibt. Somit kann sich die Zone 11 bei einer Kraftauswirkung resultierend aus steigendem Druck innerhalb des zweiten Behälters 7 relativ zu dem umgebenden Bereich ausdehnen, wodurch sich das Volumen des zweiten Behälters 7 relativ zu dem Volumen des ersten Behälters 4 vergrößert. Dies hat den Vorteil, dass ein größerer Anteil des Fluids 5 in den zweiten Behälter 7 fließen kann, obwohl das Volumen des zweiten Behälters 7 bei geschlossenem Deckel 6 möglichst klein gehalten wird, um bei dem Transport der Behältervorrichtung 1 so weit wie möglich Platz einzusparen. Die Zone 11 kann in Form eines Balgs oder als Leporello-Faltung ausgebildet sein. Die Zone 11 kann auch aus einem anderen Werkstoff bestehen als der Werkstoff, der den Bereich dieser Zone 11 umgibt.

Zusätzlich befindet sich eine fluidabsorbierende Vorrichtung 12 innerhalb des zweiten Behälters 7. Die fluidabsorbierende Vorrichtung 12 kann das Fluid 5 zumindest teilweise aufnehmen, sobald das Fluid 5 von dem ersten Behälter 4 in den zweiten Behälter 7 fließt. Das Fluid 5 ist in dem zweiten Behälter 7 mittels der fluidabsorbierenden Vorrichtung 12 zumindest teilweise gespeichert, wodurch das Fluid 5 mindestens zum Teil gebunden ist und nicht mehr im zweiten Behälter 7 frei beweglich ist. Hierdurch verringert sich die Menge an Fluid 5, die bei einer räumlichen Verlagerung, wie zum Beispiel einem Neigen des zweiten Behälters 7, aus dem zweiten Behälter 7 auslaufen kann. Somit reduziert sich die Wahrscheinlichkeit, dass es zu einem Befeuchten der Umgebung der Behältervorrichtung 1 kommt, wenn die Behältervorrichtung 1 versehentlich umgestoßen wird.

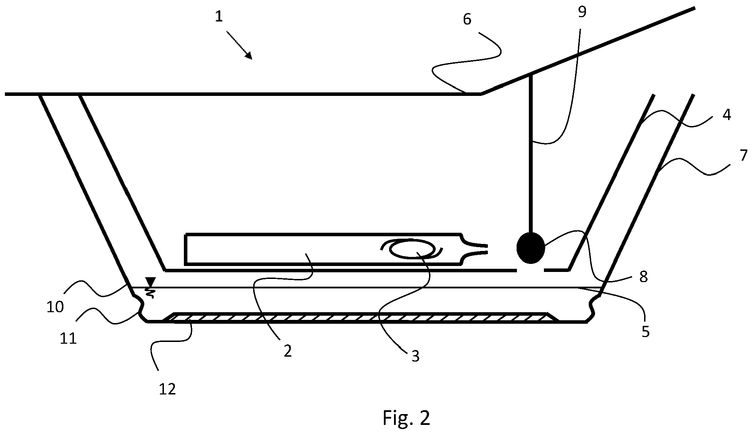

Wie oben bereits erwähnt ist, kann das Fluid 5 aus dem ersten Behälter 4 in den zweiten Behälter 7 fließen. Für diesen Zweck ist der Deckel 6 mittels einer Kopplungsvorrichtung 9 mit dem fluiddichten Verschluss 8 gekoppelt. Sobald der Deckel 6 von dem ersten Behälter 4 gelöst wird, wird der Verschluss 8 geöffnet. Der Verschluss 8 gelangt also durch Öffnen des Deckels 6 mittels der Kopplungsvorrichtung 9 von einer Geschlossen-Position in eine Offen-Position. Bei geöffnetem Verschluss 8 strömt das Fluid 5 von dem ersten Behälter 4 in den zweiten Behälter 7. Diese Situation ist in Fig. 2 zu erkennen.

In Fig. 2 ist eine schematische Darstellung der Querschnittsansicht von der Behältervorrichtung 1 gemäß der ersten Ausführungsform in einer Offen-Position dargestellt. Durch Öffnen des Deckels 6 wird der Verschluss 8 mittels der Kopplungsvorrichtung 9 geöffnet und das Fluid 5 fließt von dem ersten Behälter 4 in den zweiten Behälter 7, sodass sich anschließend kein Fluid 5 innerhalb des ersten Behälters 4 befindet. In dieser Situation kann der ophthalmologische Injektor 2 der Behältervorrichtung 1 entnommen werden, ohne dass der Benutzer mit dem Fluid 5 in Berührung kommt. Das Fluid 5 kann in dem zweiten Behälter 7 durch die fluidabsorbierende Vorrichtung 12 zumindest teilweise aufgenommen werden, wodurch die Menge an frei bewegbarem Fluid 5 verringert wird.

Außerdem entsteht bei dieser Ausführungsform durch Öffnen des Deckels 6 ein Druckausgleich zwischen dem ersten Behälter 4 und dem zweiten Behälter 7. Bevor der Deckel 6 geöffnet wird, ist der Druck innerhalb des zweiten Behälters 7 geringer als der Druck innerhalb des ersten Behälters 4. Wird dann der Deckel 6 geöffnet, dehnt sich die an der Wand 10 des zweiten Behälters 7 befindliche Zone 11, welche eine niedrigere Federkonstante als der diese Zone 11 umgebende Bereich der Wand 10 aufweist, aus, sodass sich das Volumen des zweiten Behälters 7 relativ zu dem Volumen des ersten Behälters 4 vergrößert.

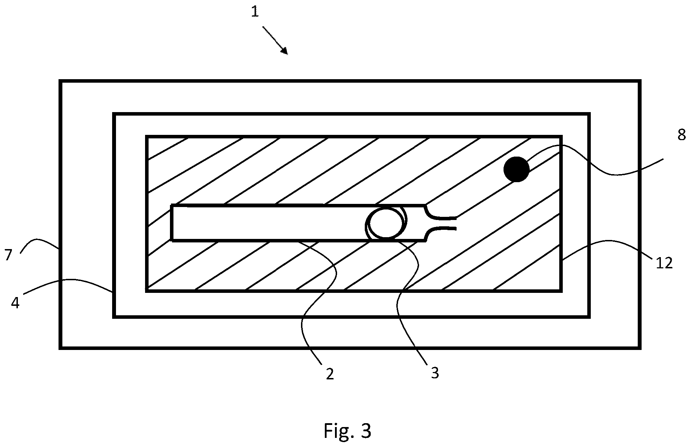

In Fig. 3 ist eine schematische Darstellung einer Draufsicht von der Behältervorrichtung 1 gemäß der in Fig. 1 gezeigten ersten Ausführungsform dargestellt. Es ist gezeigt, dass sich bei der ersten Ausführungsform der erste Behälter 4 in dem zweiten Behälter 7 befindet, wodurch die Behältervorrichtung 1 relativ kompakt ausgeführt ist. Der Injektor 2 befindet sich bevorzugt fixiert innerhalb des ersten Behälters 4, wodurch ein sicherer Transport der Behältervorrichtung 1 und damit der Intraokularlinse 3 sichergestellt ist. Bei geöffnetem Deckel 6, also wenn sich der Verschluss 8 in der Offen-Position befindet, ist die Intraokularlinse 3 nicht mehr von dem Fluid 5 umgeben und der Injektor 2 kann aus der Behältervorrichtung 1 entnommen werden, ohne dass der Benutzer in das Fluid 5 greifen muss. Zusätzlich ist die fluidabsorbierende Vorrichtung 12 in dem zweiten Behälter 7 dargestellt. Weiter ist gezeigt, dass die Grundfläche des fluiddichten Verschlusses 8 eine Größe aufweist, welche etwa 0,4 % der Grundfläche der Behältervorrichtung 1 besitzt.

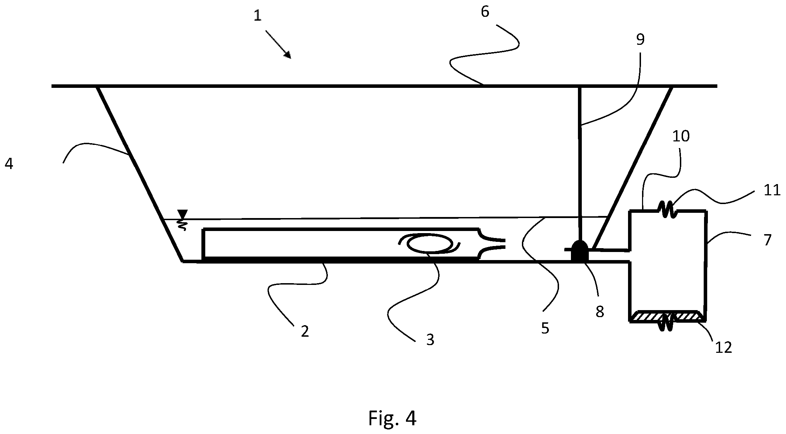

Fig. 4 zeigt eine schematische Darstellung einer Querschnittsansicht von der Behältervorrichtung 1 gemäß einer zweiten Ausführungsform in einer Geschlossen-Position. Zwar weist die Behältervorrichtung 1 ebenfalls einen ersten Behälter 4 und einen zweiten Behälter 7 auf, welche miteinander gekoppelt, also miteinander verbunden sind, jedoch befindet sich der erste Behälter 4 in der zweiten Ausführungsform anders als bei der ersten Ausführungsform nicht in dem zweiten Behälter 7. In dieser Ausführungsform verschließt der Deckel 6 nur den ersten Behälter 4. Im Gegensatz zu der ersten Ausführungsform verschließt der Deckel 6 nicht den zweiten Behälter 7 und ist mit dem zweiten Behälter 7 auch nicht lösbar verbunden.

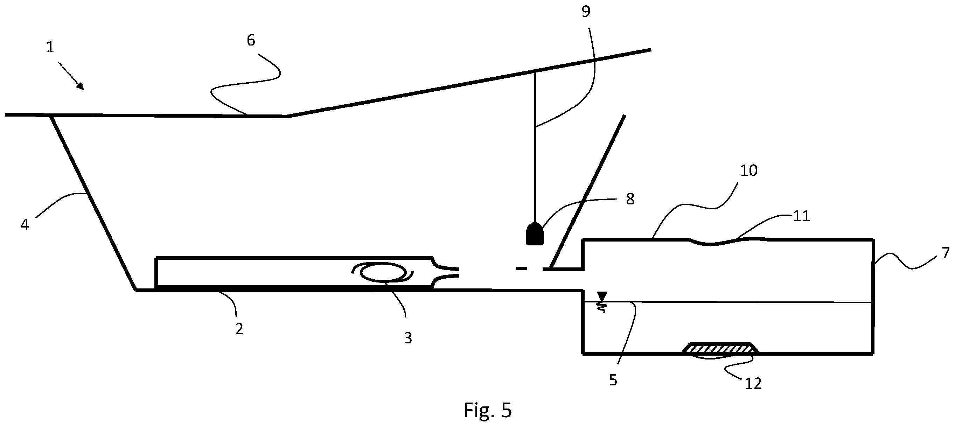

Der ophthalmologische Injektor 2, welcher die Intraokularlinse 3 enthält und von dem Fluid 5 umgeben ist, befindet sich in dem ersten Behälter 4. In der Geschlossen-Position, in welcher der mit dem Deckel 6 verbundene fluiddichte Verschluss 8 so eingerichtet ist, dass das Fluid 5 nicht von dem ersten Behälter 4 in den zweiten Behälter 7 strömen kann, herrschen unterschiedliche Drücke in dem ersten Behälter 4 und in dem zweiten Behälter 7. Präziser formuliert liegt in dem ersten Behälter 4 ein höherer Druck vor als in dem zweiten Behälter 7, sodass durch Öffnen des Deckels 6, wodurch der Verschluss 8 mittels der Kopplungsvorrichtung 9 von der Geschlossen-Position in die Offen-Position übergeht, das Fluid 5 schneller in den zweiten Behälter 7 gelangt. Diese Offen-Position ist in Fig. 5 dargestellt.

Fig. 5 zeigt eine schematische Darstellung der Querschnittsansicht von der Behältervorrichtung 1 gemäß der zweiten Ausführungsform in der Offen-Position. Durch Öffnen des Deckels 6 findet ein Druckausgleich zwischen dem ersten Behälter 4 und dem zweiten Behälter 7 statt, wodurch sich die Zone 11 der Wand 10, welche eine niedrigere Federkonstante aufweist als ein diese Zone 11 umgebender Bereich der Wand 10, ausdehnt und sich das Volumen des zweiten Behälters 7 vergrößert. Das Fluid 5 fließt in den zweiten Behälter 7 und wird von der fluidabsorbierenden Vorrichtung 12 zumindest teilweise aufgenommen.

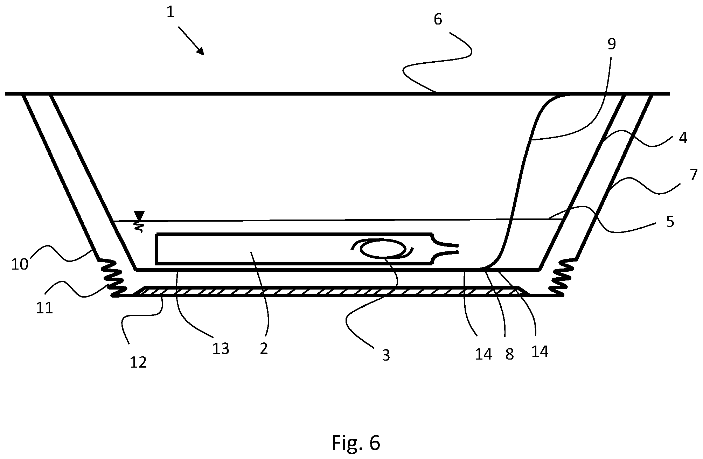

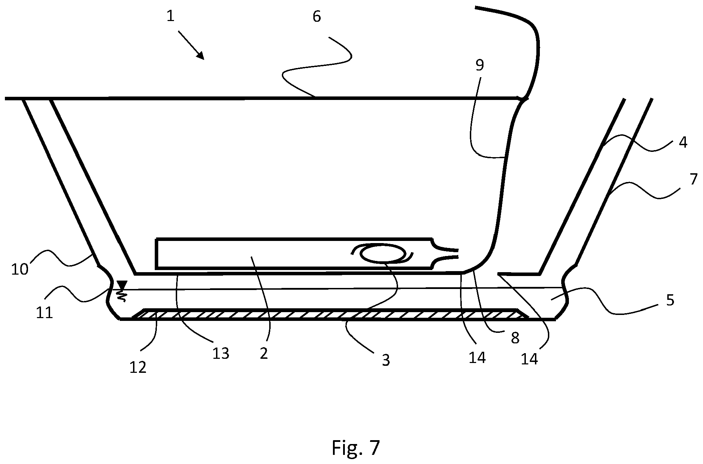

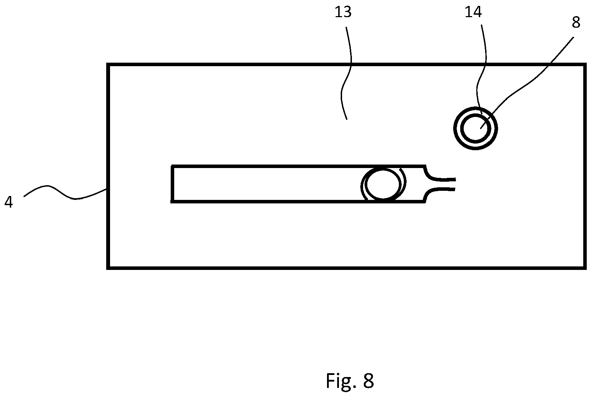

In Fig. 6 ist eine schematische Darstellung einer Querschnittsansicht von der Behältervorrichtung 1 gemäß einer dritten Ausführungsform in einer Geschlossen-Position dargestellt. In der dritten Ausführungsform ist die Kopplungsvorrichtung 9 ein Teil des ersten Behälters 4, welcher mit dem Deckel 6 verbunden ist. Auch der fluiddichte Verschluss 8 ist ein Teil des ersten Behälters 4. Eine Wand des ersten Behälters 4, welche hier eine Bodenwand ist, weist einen ersten Wandbereich 13 mit einer ersten Dicke und einen zweiten Wandbereich 14 mit einer zweiten Dicke auf. Der fluiddichte Verschluss 8 ist in dieser Ausführungsform zumindest teilweise durch einen Teil der Bodenwand des ersten Behälters 4 ausgebildet, wobei der Verschluss 8 durch den zweiten Wandbereich 14 zumindest teilweise begrenzt ist. Die zweite Dicke ist kleiner als die erste Dicke, wodurch der Verschluss 8 eine Sollbruchstelle aufweist. Der zweite Wandbereich 14 kann als RingSegment, als umlaufender Kreisring, siehe Fig. 8, oder als Kreisfläche ausgebildet sein. Ist der zweite Wandbereich 14 als Kreisfläche ausgebildet, so ist diese Kreisfläche der Verschluss 8. Wenn der Deckel 6 geöffnet wird, überträgt sich die Zugkraft mittels der Kopplungsvorrichtung 9 auf den Verschluss 8, welcher an dem zweiten Wandbereich 14 des ersten Behälters 4 aufbricht, welcher die zweite Dicke aufweist, sodass der Verschluss 8 von einer Geschlossen-Position in eine Offen-Position gelangt. Diese Offen-Position ist in Fig. 7 dargestellt. Nach dem Aufbrechen an der Sollbruchstelle kann der Verschluss 8 nicht mehr in seine Geschlossen-Position zurückgelangen. Die Behältervorrichtung 1 ist somit nur für einen einmaligen Gebrauch vorgesehen.

Fig. 7 zeigt eine schematische Darstellung der Querschnittsansicht von der Behältervorrichtung 1 gemäß der dritten Ausführungsform in der Offen-Position. Durch Öffnen des Deckels 6 transferiert sich die Zugkraft mittels der Kopplungsvorrichtung 9 auf den fluiddichten Verschluss 8. Hierdurch bricht der Verschluss 8 an dem zweiten Wandbereich 14 des ersten Behälters 4 auf, welcher die zweite Dicke aufweist, sodass der Verschluss 8 von einer Geschlossen-Position in eine Offen-Position gelangt. Hierdurch ist sichergestellt, dass das Fluid 5 von dem ersten Behälter 4 in den zweiten Behälter 7 fließen kann, sodass sich anschließend kein Fluid 5 innerhalb des ersten Behälters 4 befindet. Wenn das Fluid 5 in dem zweiten Behälter 7 aufgenommen ist, kann der ophthalmologische Injektor 2 aus dem ersten Behälter 4 entnommen werden, ohne dass der Benutzer mit dem Fluid 5 in Berührung kommt. Das Fluid 5 kann in dem zweiten Behälter 7 durch die fluidabsorbierende Vorrichtung 12 zumindest teilweise aufgenommen werden, wodurch die Menge an frei bewegbarem Fluid 5 verringert wird.

Vorzugsweise entsteht durch Öffnen des Deckels 6 ein Druckausgleich zwischen dem ersten Behälter 4 und dem zweiten Behälter 7. Bevor der Deckel 6 geöffnet wird, ist der Druck innerhalb des zweiten Behälters 7 geringer als der Druck innerhalb des ersten Behälters 4. Wird dann der Deckel 6 geöffnet, kann das Fluid 5 schnell und zuverlässig in den zweiten Behälter 7 abfließen. An der Wand 10 des zweiten Behälters 7 ist bevorzugt eine Zone 11 vorgesehen, welche eine niedrigere Federkonstante als der diese Zone 11 umgebende Bereich der Wand 10 aufweist, sodass sich das Volumen des zweiten Behälters 7 relativ zu dem Volumen des ersten Behälters 4 vergrößert, wenn das Fluid 5 vom ersten Behälter 4 in den zweiten Behälter 7 fließt.

### Bezugszeichenliste

- 1: Behältervorrichtung
- 2: Ophthalmologischer Injektor
- 3: Intraokularlinse
- 4: Erster Behälter
- 5: Fluid
- 6: Deckel
- 7: Zweiter Behälter
- 8: Fluiddichter Verschluss
- 9: Kopplungsvorrichtung
- 10: Wand
- 11: Zone
- 12: Fluidabsorbierende Vorrichtung
- 13: Erster Wandbereich des ersten Behälters
- 14: Zweiter Wandbereich des ersten Behälters

## Patentansprüche

1. Behältervorrichtung (1), aufweisend:
- einen ophthalmologischen Injektor (2), welcher eine Intraokularlinse (3) aufweist,
- einen ersten Behälter (4), der zumindest teilweise mit einem Fluid (5) gefüllt ist, wobei die Intraokularlinse (3) von dem Fluid (5) umgeben ist, und der erste Behälter (4) mit einem Deckel (6) versehen ist, welcher den ersten Behälter (4) fluiddicht verschließt und lösbar mit dem ersten Behälter (4) verbunden ist,
- einen zweiten Behälter (7), der mit dem ersten Behälter (4) gekoppelt ist,
**dadurch gekennzeichnet, dass** zwischen dem ersten Behälter (4) und dem zweiten Behälter (7) ein fluiddichter Verschluss (8) vorgesehen ist,
wobei der Deckel (6) mittels einer Kopplungsvorrichtung (9) mit dem Verschluss (8) gekoppelt ist, und bei einem Lösen des Deckels (6) von dem ersten Behälter (4) der Verschluss (8) mittels der Kopplungsvorrichtung (9) eingerichtet ist, von einer Geschlossen-Position in eine Offen-Position zu gelangen, sodass das Fluid (5) von dem ersten Behälter (4) in den zweiten Behälter (7) strömen kann.

2. Behältervorrichtung (1) nach Anspruch 1, wobei der Verschluss (8) eine Fläche aufweist und die Fläche des Verschlusses (8) eine Größe in einem Bereich von 50 % bis 20 % der Fläche einer Wand der Behältervorrichtung (1) besitzt, oder 20 % bis 10 % der Fläche einer Wand der Behältervorrichtung (1) besitzt, oder eine Größe in einem Bereich von 10 % bis 1 % der Fläche einer Wand der Behältervorrichtung (1) besitzt, oder eine Größe in einem Bereich von 1 % bis 0,2 % der Fläche einer Wand der Behältervorrichtung (1) besitzt.

3. Behältervorrichtung (1) nach Anspruch 1 oder 2, wobei der Verschluss (8) ein Einwegeventil ist.

4. Behältervorrichtung (1) nach einem der vorherigen Ansprüche, wobei in dem ersten Behälter (4) ein erster Druck vorliegt und in dem zweiten Behälter (7) ein zweiter Druck vorliegt, wobei der zweite Druck niedriger als der erste Druck ist.

5. Behältervorrichtung (1) nach einem der vorherigen Ansprüche, wobei mindestens eine Wand (10) des zweiten Behälters (7) mindestens eine Zone (11) aufweist, welche eine niedrigere Federkonstante als ein diese Zone (11) umgebender Bereich der Wand (10) aufweist.

6. Behältervorrichtung (1) nach einem der vorherigen Ansprüche, wobei der zweite Behälter (7) eine fluidabsorbierende Vorrichtung (12) aufweist.

7. Behältervorrichtung (1) nach einem der vorherigen Ansprüche, wobei der Deckel (6) den zweiten Behälter (7) fluiddicht verschließt und lösbar mit dem zweiten Behälter (7) verbunden ist.

8. Behältervorrichtung (1) nach einem der vorherigen Ansprüche, wobei die Kopplungsvorrichtung (9) ein Teil des ersten Behälters (4) ist.

## Claims

1. Container device (1), having:
- an ophthalmic injector (2) which has an intraocular lens (3),
- a first container (4), which is at least partially filled with a fluid (5), wherein the intraocular lens (3) is surrounded by the fluid (5), and the first container (4) is provided with a lid (6) which closes the first container (4) in a fluid-tight manner and which is releasably connected to the first container (4),
- a second container (7), which is coupled to the first container (4),
**characterized in that** a fluid-tight closure (8) is provided between the first container (4) and the second container (7),
wherein the lid (6) is coupled to the closure (8) by means of a coupling device (9), and, when the lid (6) is released from the first container (4), the closure (8) is configured, by means of the coupling device (9), to pass from a closed position to an open position, such that the fluid (5) can flow from the first container (4) into the second container (7).

2. Container device (1) according to Claim 1, wherein the closure (8) has a surface area, and the surface area of the closure (8) has a size in a range of 50% to 20% of the surface area of a wall of the container device (1), or 20% to 10% of the surface area of a wall of the container device (1), or has a size in a range of 10% to 1% of the surface area of a wall of the container device (1), or has a size in a range of 1% to 0.2% of the surface area of a wall of the container device (1).

3. Container device (1) according to Claim 1 or 2, wherein the closure (8) is a one-way valve.

4. Container device (1) according to any of the preceding claims, wherein in the first container (4) there is a first pressure and in the second container (7) there is a second pressure, the second pressure being lower than the first pressure.

5. Container device (1) according to any of the preceding claims, wherein at least one wall (10) of the second container (7) has at least one zone (11) which has a lower spring constant than a region of the wall (10) surrounding this zone (11).

6. Container device (1) according to any of the preceding claims, wherein the second container (7) has a fluid-absorbing device (12).

7. Container device (1) according to any of the preceding claims, wherein the lid (6) closes the second container (7) in a fluid-tight manner and is releasably connected to the second container (7).

8. Container device (1) according to any of the preceding claims, wherein the coupling device (9) is part of the first container (4).

## Revendications

1. Dispositif (1) à récipients, comprenant :
- un injecteur ophtalmologique (2) comprenant une lentille intraoculaire (3),
- un premier récipient (4) qui est au moins partiellement rempli d'un fluide (5), la lentille intraoculaire (3) étant entourée par le fluide (5), et le premier récipient (4) étant pourvu d'un couvercle (6) qui ferme le premier récipient (4) de manière étanche aux fluides et qui est relié de manière amovible au premier récipient (4),
- un deuxième récipient (7) qui est relié au premier récipient (4),
**caractérisé en ce qu'**un obturateur étanche aux fluides (8) est prévu entre le premier récipient (4) et le deuxième récipient (7),
le couvercle (6) étant relié à l'obturateur (8) au moyen d'un dispositif de liaison (9) et l'obturateur (8) étant conçu pour passer, lorsque le couvercle (6) est détaché du premier récipient (4), au moyen du dispositif de liaison (9), d'une position fermée à une position ouverte, de sorte que le fluide (5) soit apte à s'écouler du premier récipient (4) dans le deuxième récipient (7).

2. Dispositif (1) à récipients selon la revendication 1, dans lequel l'obturateur (8) présente une surface et la surface de l'obturateur (8) a une taille comprise dans une gamme allant de 50 % à 20 % de la surface d'une paroi du dispositif (1) à récipients, ou a une taille comprise dans une gamme allant de 20 % à 10 % de la surface d'une paroi du dispositif (1) à récipients, ou a une taille comprise dans une gamme allant de 10 % à 1 % de la surface d'une paroi du dispositif (1) à récipients, ou a une taille comprise dans une gamme allant de 1 % à 0,2 % de la surface d'une paroi du dispositif (1) à récipients.

3. Dispositif (1) à récipients selon la revendication 1 ou la revendication 2, dans lequel l'obturateur (8) est une vanne unidirectionnelle.

4. Dispositif (1) à récipients selon l'une des revendications précédentes, dans lequel une première pression est présente dans le premier récipient (4) et une deuxième pression est présente dans le deuxième récipient (7), la deuxième pression étant inférieure à la première pression.

5. Dispositif (1) à récipients selon l'une des revendications précédentes, dans lequel au moins une paroi (10) du deuxième récipient (7) comporte au moins une zone (11) présentant une constante d'élasticité inférieure à celle d'une zone de la paroi (10) entourant cette zone (11).

6. Dispositif (1) à récipients selon l'une des revendications précédentes, dans lequel le deuxième récipient (7) comporte un dispositif d'absorption de fluide (12).

7. Dispositif (1) à récipients selon l'une des revendications précédentes, dans lequel le couvercle (6) ferme le deuxième récipient (7) de manière étanche aux fluides et est relié de manière amovible au deuxième récipient (7).

8. Dispositif (1) à récipients selon l'une des revendications précédentes, dans lequel le dispositif de liaison (9) est une partie du premier récipient (4).
